# EUROPEAN PATENT APPLICATION

(11) **EP 4 785 949 A1**
(43) Date of publication of application: **05.08.2026**
(21) Application number: 24871994.0
(22) Date of filing: 18.09.2024
(51) Int. Cl.: A61K 36/82, A23L 33/105, A61K 36/06, A61P 43/00

(54) **AUTOPHAGY ACTIVATOR**

(30) Priority: 25.09.2023 JP 2023161522
(71) Applicant: Kawachikinhondo Co., Ltd., Kirishima-shi, Kagoshima 899-6404 (JP)
(72) Inventor: YAMAMOTO Bunsei, Kirishima-shi, Kagoshima 899-6404 (JP); TATEBE Hisashi, Suita-shi, Osaka 565-0871 (JP); FUKUDA Kanako, Suita-shi, Osaka 565-0871 (JP); SHOJI Shisako, Suita-shi, Osaka 565-0871 (JP); SHOSHIHARA Masako, Suita-shi, Osaka 565-0871 (JP); ISHIDO Miwako, Suita-shi, Osaka 565-0871 (JP)
(74) Representative: Mewburn Ellis LLP
(86) International application number: PCT/JP2024/033255
(87) International publication number: WO 2025/070202

(57) **Abstract**

Provided is an autophagy activator that is capable of accelerating autophagy activity and also has good safety.

The autophagy activator of the present invention contains a koji mold fermentation product of tea leaves or an extract of the koji mold fermentation product of tea leaves.

## Description

### TECHNICAL FIELD

The present invention relates to an autophagy activator, containing a koji mold fermentation product of tea leaves or an extract thereof.

### BACKGROUND ART

Autophagy is a system to decompose and recycle unnecessary proteins, organelles and the like in cells in eukaryotes. Autophagy plays not only a role to acquire nutrient sources such as amino acids by decomposing proteins and the like but also an important role to maintain cell homeostasis by purifying the inside of cells such as removing damaged organelles and promoting the turnover of old cells.

In recent years the results of studies on autophagy have revealed that autophagy dysfunction is a main cause for neurodegenerative diseases such as Parkinson's disease and Alzheimer's disease, kidney diseases and the like (see Patent Literatures 1 and 2). According to this, harmful proteins and abnormal organelles are accumulated in cells by suppression of autophagy activity, which is a cause to develop these diseases. It is known that suppression and reduction of autophagy activity are involved in various diseases such as lifestyle-related diseases and carcinogenesis in addition to the diseases described above, and moreover are a cause to develop aging-associated diseases.

Meanwhile, recently, traditional Japanese koji fermentation food products using koji, such as sake, shochu, amazake, miso and soy sauce, has been re-recognized for value and popularity of koji is increasing. "Koji" means a product obtained by steaming rice, rice bran, wheat, bean or the like, and then allowing koji molds to proliferate therein. Among them, koji obtained by allowing koji mold to proliferate in rice is referred to as "rice koji (malted rice)". Koji molds are known to produce many types of enzymes, by the functions of which fermentation food products can be produced. Studies on new function of koji molds and the development of applications based thereon have been advanced in fields not limited to those aforementioned. Patent Literature 3, for example, reports that a koji mold fermentation product obtained by fermentation with a koji mold has the effect of improving male infertility and Patent Literature 4 reports that a koji mold fermentation product obtained by fermentation with a koji mold has the effect of hair restoration and growth.

### CITATION LIST

### PATENT LITERATURE

Patent Literature 1: WO2023/276828
Patent Literature 2: WO2022/054927
Patent Literature 3: WO2020/008821
Patent Literature 4: WO2020/226022

### SUMMARY OF INVENTION

### TECHNICAL PROBLEM

As described above, reduction and suppression of autophagy activity can be a cause to develop various diseases. Therefore, in order to prevent and treat the development of these diseases and achieve healthy longevity, an autophagy activator which can accelerate autophagy activity has been demanded. An autophagy activator with high safety which can be taken safely on a daily basis as medicine or functional food has been further expected.

Patent Literatures 3 and 4 also each describe a koji mold fermentation product obtained by fermenting grain and tea leaves with a koji mold; however, the use of this koji mold fermentation product to accelerate autophagy activity has not yet been studied, and thus, effectiveness of the products is not known.

Therefore, the present invention has been made in view of the above-described points and an object thereof is to provide an autophagy activator which can accelerate autophagy activity and has good safety.

### SOLUTION TO PROBLEM

In order to solve the above problems, the autophagy activator of the present invention contains a koji mold fermentation product of tea leaves or an extract of this koji mold fermentation product of tea leaves. The koji mold fermentation product and the extract thereof have a good autophagy activity accelerating action and are also useful to treat, prevent or improve diseases caused by suppression of autophagy activity. In addition, the koji mold fermentation product of tea leaves is obtained from tea leaves and a koji mold and thus has high safety.

The above-described koji mold is preferably yellow koji mold or black koji mold. Because of this, as the koji mold causing a koji mold fermentation product, an active ingredient of the autophagy activator of the present invention, a koji mold having a good autophagy activity accelerating action is selected. In addition, the yellow koji mold and black koji mold are koji molds used to produce fermented food and thus koji mold fermentation products obtained from these koji molds have high safety and can be taken on a daily basis.

In addition, the koji mold in the above-described koji mold fermentation product is a viable koji mold. Therefore, an autophagy activator having a good autophagy activity accelerating action is obtained.

In addition, the extract of the koji mold fermentation product in the present invention is preferably a water extract or a hydrous alcohol extract of the koji mold fermentation product. Because of this, an autophagy activator having a good autophagy activating effect is obtained safely and easily at a low cost.

In addition, the method for producing an autophagy activator of the present invention has a step of seeding a koji mold in tea leaves or a material derived from tea leaves as a fermentation raw material, and a step of fermenting the fermentation raw material with this koji mold to obtain a koji mold fermentation product. Because of this, an autophagy activator which has a good autophagy activity accelerating action and is also useful to treat, prevent or improve diseases caused by suppression of autophagy activity is obtained.

In addition, in the method for producing an autophagy activator of the present invention, the above-described koji mold is preferably yellow koji mold or black koji mold. Because of this, as the koji mold causing a koji mold fermentation product, an active ingredient in the autophagy activator of the present invention, a suitable koji mold is selected. In addition, the yellow koji mold and black koji mold are koji molds used to produce fermented food and thus an autophagy activator with high safety which can be taken on a daily basis can be obtained.

In addition, the method for producing an autophagy activator of the present invention preferably has a step of extracting the obtained koji mold fermentation product with water or a hydrous alcohol to obtain an extract of the koji mold fermentation product. Because of this, an autophagy activator having a good autophagy activating effect is obtained safely and easily at a low cost.

In addition, in the method for producing an autophagy activator of the present invention, the koji mold in the koji mold fermentation product is a viable koji mold. Because of this, an autophagy activator having a good autophagy activity accelerating action can be produced.

### ADVANTAGEOUS EFFECT OF INVENTION

According to the present invention, it is possible to provide an autophagy activator having good effects as described below:
(1) having a good autophagy activity accelerating action;
(2) being able to be used to treat, prevent or improve diseases caused by suppression or reduction of autophagy activity;
(3) having high safety because a fermentation product obtained from tea leaves popular as "tea" and a koji mold popular as fermented food is an active ingredient; and
(4) being able to also obtain an autophagy activator of liquid medicine or drink type only by extracting the koji mold fermentation product with water or a hydrous alcohol.

### BRIEF DESCRIPTION OF DRAWINGS

FIG. 1 is a flowchart schematically showing the method for producing a koji mold fermentation product of tea leaves and an extract thereof according to an embodiment of the present invention.
FIG. 2 is a graph showing the results of the tfLC3 assay to evaluate the autophagy activity accelerating action in Example 3.

### DESCRIPTION OF EMBODIMENT

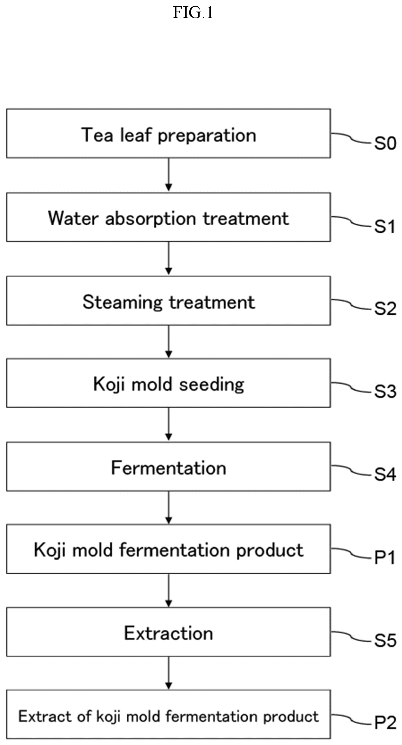

First, the method for producing a koji mold fermentation product P1 of tea leaves contained in the autophagy activator of the present invention will be described with reference to FIG. 1.

As shown in FIG. 1, the method for producing a koji mold fermentation product P1 of tea leaves according to the embodiment of the present invention is schematically configured from a step S0 of preparing tea leaves, as a fermentation raw material, a step S1 of adding water to the tea leaves for water absorption, a step S2 of steaming the tea leaves, a step S3 of seeding a koji mold in the steamed tea leaves, and a step S4 of carrying out fermentation.

### (Tea leaf preparation)

First, the step S0 of preparing tea leaves, a fermentation raw material, shown in FIG. 1 will be described. As the fermentation raw material in the present invention, tea leaves from the tea plant (Camellia sinensis) can be used. The tea leaves may include not only leaves of tea but also stems, branches and the like collected from the tea plant. As tea leaves used as a raw material for the koji mold fermentation product of the present invention, raw tea leaves in the state of being collected are suitably used; however, tea leaves refrigerated after collected, tea leaves heated after collected, tea leaves oxidized and fermented with an oxidase contained in tea leaves after collected, and tea obtained after tea manufacturing steps may be used. In addition, used tea leaves after extracting these green teas, oolong teas, black teas or the like can be also used. In order to enhance the efficiency of a steaming treatment, a subsequent step, and the culturing efficiency of a koji mold, tea leaves and tea stems may be crushed to a certain size so that the size will not be too large.

### (Water absorption treatment)

Next, the water absorption treatment step S1 will be described. In this step, water absorption is carried out by adding water to the tea leaves or immersing the tea leaves in water. The amount of water to be absorbed is not particularly limited and is specifically adjusted to fall within the range of 20 to 60% and preferably 30 to 50%, in terms of water content of the tea leaves.

### (Steaming treatment)

Next, the steaming treatment step S2 will be described. The tea leaves which were allowed to absorb water in the above-described step are placed in a steamer, which is covered with a lid and heating is carried out by heating the steamer to apply water vapor to the tea leaves. The time for the steaming treatment is preferably about 30 minutes to 120 minutes, more preferably about 45 minutes to 90 minutes and particularly preferably about 60 minutes. By the steaming treatment, undesirable bacteria present in the raw materials are killed, with the result that fermentation in the later step can be carried out by a dominant species, a koji mold. Even if fresh tea leaves are used as a fermentation raw material, if the steaming treatment is carried out, undesirable bacteria present in tea leaves are killed, and simultaneously, oxidase contained in the tea leaves are inactivated, with the result that the koji mold can be efficiently proliferated in tea leaves in the step later carried out. The steaming treatment can be also carried out using, in addition to the steamer, a steaming device which can introduce steam in the device. The tea leaves after completion of steaming are taken out of the steamer, spread evenly on a table and cooled up to about 30 to 40°C. By doing this, the tea leaves can be ready for inoculation with the koji mold.

### (Koji mold seeding)

Next, the step S3 of seeding (inoculating) a koji mold in the tea leaves will be described. In this step, the koji mold is seeded in the tea leaves cooled after the above-described steaming step. In the present invention, the koji mold refers to a microorganism for use in mainly producing koji fermentation food; more specifically, examples of the koji mold include yellow koji mold, black koji mold, and white koji mold. Among these, yellow koji mold indicates a mold group of the genus Aspergillus forming yellow or yellow-green conidia and are microorganisms used to produce mainly refined sake, miso, soy sauce, and the like. Specific examples thereof include, but not limited to, Aspergillus oryzae, Aspergillus oryzae var. kawachii (Kawachi yellow koji mold), Aspergillus sojae and the like. Furthermore, black koji mold indicates a mold group of the genus Aspergillus forming black or dark brown conidia (a kind of asexual spore) used to produce distilled liquors such as awamori in Okinawa and sweet potato shochu in Kagoshima. Specific examples thereof include, but not limited to, Aspergillus luchuensis (Aspergillus awamori), Aspergillus luchuensis var. kawachii (Kawachi black koji mold), Aspergillus saitoi, Aspergillus inuii, Aspergillus usamii, Aspergillus aureus and the like. In addition, white koji mold indicates a mold group of the genus Aspergillus forming white-yellow conidia, and specific examples thereof include Aspergillus kawachii (Aspergillus luchuensis mut. kawachii). In the present invention, yellow koji mold or black koji mold is preferably used and yellow koji mold is further preferably used from the viewpoint of a good autophagy activity accelerating effect. Among these, Kawachi yellow koji mold (Aspergillus oryzae var. kawachii) as yellow koji mold, Aspergillus oryzae or Aspergillus sojae, Kawachi black koji mold (Aspergillus luchuensis var. kawachii) as black koji mold, Aspergillus luchuensis or Aspergillus inuii and combinations thereof are suitably used, and Kawachi yellow koji mold (Aspergillus oryzae var. kawachii) is particularly preferably used. These koji molds can be seeded, for example, by mixing a koji mold in tea leaves put in a sterilized bag and stirring them. Spores of the koji mold are preferably seeded, and the spores of the koji mold are preferably added and inoculated at half a million spores or more and preferably one million spores or more with respect to 1 g of a fermentation raw material (dry weight). When the number of spores contained in 1 g of seed malt is two billion, for example, it is only needed to add about 0.5 g (0.05 w/w%) of seed malt with respect to 1 kg of the tea leaves. After seeding the koji mold in the tea leaves, the koji mold is preferably dispersed in all the tea leaves by stirring well.

### (Fermentation)

Next, the step S4 of carrying out fermentation by culturing the koji mold seeded in the tea leaves will be described. In this step, the koji mold is proliferated in the tea leaves in which the koji mold has been seeded. First, the tea leaves to which the koji mold has been added are put in a culturing room maintained at about 30°C. As time passes, fermentation proceeds and the temperature of the tea leaves increases. However, proliferation of the koji mold is usually difficult over 40°C and thus the temperature is reduced by ventilation. The amount of ventilation is adjusted so that the temperature of the tea leaves is maintained at 30°C to 42°C and preferably 30°C to 40°C. It should be noted that the fermentation temperature is appropriately adjusted depending on the types of koji mold used for fermentation. Specifically, when yellow koji mold is selected as the koji mold, the fermentation temperature is adjusted so that the temperature of tea leaves will be 30°C to 35°C for 12 to 30 hours from the start of culturing such as by adjusting the room temperature of a fermentation room, as an example. After that, the fermentation temperature is adjusted slightly higher so that the temperature will be 35°C to 40°C by adjusting the room temperature or the like similarly, and the time point when the koji mold is cultured for about 1 to 4 days in total from the start of culturing, preferably 36 to 72 hours and further preferably 40 to 60 hours is considered completion of a koji mold fermentation product P1. On the other hand, when black koji mold or white koji mold is selected as the koji mold, the fermentation temperature is adjusted so that the temperature of tea leaves will be 35°C to 40°C for 12 to 30 hours from the start of culturing such as by adjusting the room temperature of a fermentation room, as an example. After that, the fermentation temperature is adjusted slightly lower so that the temperature will be 30°C to 35°C by adjusting the room temperature or the like similarly, and the time point when the koji mold is cultured for about 1 to 4 days in total from the start of culturing, preferably 36 to 72 hours and further preferably 40 to 60 hours is considered completion of a koji mold fermentation product P1.

It should be noted that the above-described steps S1 to S4 of producing a koji mold fermentation product P1 can be also carried out by a machine which can carry out washing of tea leaves, water absorption, steaming, seeding of a koji mold and fermentation (koji making) in a single device (e.g., a drum type automatic koji making device).

### (Koji mold fermentation product)

The obtained koji mold fermentation product P1 is so-called "koji", that is a proliferated koji mold in the tea leaves, and the koji mold is contained in the koji mold fermentation product P1 as a viable koji mold (including the state of spores). This koji mold fermentation product P1 has a good autophagy activating effect and is useful to treat, prevent or improve diseases caused by suppression of autophagy activity. The koji mold fermentation product P1 obtained by the above-described steps is in a state containing a certain amount of moisture, and moisture can be removed by natural drying or low-temperature dehumidification drying so that the koji mold will not die out. In addition, the koji mold fermentation product P1 after removing moisture can be crushed into powder or granules. By reducing the moisture content of the koji mold fermentation product P1, the activity and growth of the koji mold in the koji mold fermentation product P1 can be suppressed to store the koji mold fermentation product P1. Furthermore, the koji mold fermentation product P1 can be stored for a long period of time with the koji mold viable by refrigerated storage. The autophagy activating effect as the koji mold fermentation product P1 can be effectively retained even after removing moisture.

Next, the method for producing an extract P2 of the koji mold fermentation product contained in the autophagy activator of the present invention will be described. As shown in FIG. 1, the method for producing an extract P2 of the koji mold fermentation product according to the embodiment of the present invention is schematically configured from a step S5 of adding an extraction solvent to the koji mold fermentation product P1 obtained by the steps S1 to S4 described above for extraction.

### (Extraction)

The extraction step S5 will be described in more detail. In this extraction step S5, an extraction solvent is added to the koji mold fermentation product P1 for an extraction treatment to obtain an extract P2 of the koji mold fermentation product. The extraction solvent is not particularly limited as long as a component having the autophagy activity accelerating action can be extracted from the koji mold fermentation product P1, and examples thereof include polar protic solvents such as water and alcohols, polar aprotic solvents such as dimethyl sulfoxide and acetone, and non-polar solvents such as hexane. Among these, water or an alcohol and a hydrous alcohol obtained by combining the above is preferably used as an extraction solvent from the viewpoint of extraction efficiency, safety to human bodies and easy handling. Among these, examples of the alcohol include ethanol, 1,3-butylene glycol, isopropanol, propylene glycol, glycerin and the like, and one or two or more of these can be also used in combination. As ethanol described above, furthermore, purified ethanol and also a fermented liquid obtained by alcoholic fermentation can be used. Specifically, a brewage or a distilled liquor obtained by distilling a brewage can be used and among these, a distilled liquor can be suitably used. The alcohol concentration of the hydrous alcohol is preferably 1 to 50 v/v%, more preferably 3 to 30 v/v% and particularly preferably 5 to 15 v/v%. It should be noted that in addition to the above-described water, an alcohol or a hydrous alcohol, other components such as a pH adjuster, a preservative and an extraction aid can be contained in the extraction solvent without preventing the extraction of a useful component from the koji mold fermentation product P1.

As the method for extraction from the koji mold fermentation product P1, it is preferred to add the koji mold fermentation product P1 to an extraction solvent to immerse the product P1 in the solvent and carry out extraction. When a dried product having a water content of less than 10% is used as the koji mold fermentation product P1, this is added in an amount of preferably 1 g to 300 g, more preferably 5 g to 150 g and particularly preferably 10 g to 100 g with respect to 1 L of the extraction solvent. In addition, in order to maintain the activity of various proteins and enzymes contained in the koji mold fermentation product P1, the extraction temperature is preferably about 0 to 30°C, more preferably about 2 to 20°C and particularly preferably 4 to 10°C as extraction conditions. In addition, the extraction time is preferably about two hours to 5 days, more preferably about 6 hours to three days and particularly preferably about 12 hours to 1.5 days.

The extract P2 of the koji mold fermentation product is obtained by removing residue such as by decantation, centrifugation or filtration after the extraction treatment described above. The extract P2 of the koji mold fermentation product of the present invention also includes the extract P2 itself of the koji mold fermentation product obtained by the extraction step S5 and also a concentrated liquid of the extract P2 by a concentration treatment such as vacuum concentration, a solid and powder of the extract P2 of the koji mold fermentation product by a drying treatment such as a freeze-drying treatment, and the like.

In the present invention, the activation of autophagy indicates to accelerate the autophagy activity in cells, and means that autophagy activity is more accelerated than of a control to which the autophagy activator of the present invention is not added or administered. The autophagy activity accelerating action can be evaluated, for example, by a tfLC3 assay carried out in Examples described below, but not limited thereto. More specifically, the GFP/RFP ratio value in the tfLC3 assay is preferably less than 1.00, more preferably 0.90 or less and further preferably 0.80 or less.

The autophagy activator of the present invention contains a koji mold fermentation product P1 of tea leaves fermented with the above-described koji mold or an extract P2 of the koji mold fermentation product of tea leaves as an active ingredient, and has the autophagy activity accelerating action. Therefore, the autophagy activator containing the koji mold fermentation product or the extract of the koji mold fermentation product of the present invention can be used as a drug, quasi-drug and food to treat, prevent or improve diseases caused by suppression and reduction of autophagy activity. Particularly, the food includes supplements, health food, functional food, Food for Specified Health Uses and the like. The autophagy activator of the present invention contains a fermentation product with a koji mold used such as for production of fermented food as an active ingredient and thus has high safety and can be used safely and easily regardless of age, sex, chronic diseases, and the like.

The dosage of the autophagy activator of the present invention varies depending on targeted prevention, improvement or treatment effects, administration methods, disease types and the like, and thus cannot be generally provided. However, the daily dosage by oral administration is usually about 10 to 1000 mg/kg body weight as the koji mold fermentation product P1, preferably about 20 to 500 mg/kg body weight and further preferably about 50 to 300 mg/kg body weight, which can be divided into one to three times from the start of administration and administered. It is also about 10 to 1000 mg/kg body weight as the extract P2 of the koji mold fermentation product, preferably about 20 to 500 mg/kg body weight and further preferably about 50 to 300 mg/kg body weight, which can be divided into one to three times from the start of administration and administered.

The autophagy activator of the present invention can be prepared into various forms by conventional methods which are commonly used. In this case, the autophagy activator can be formulated using additives acceptable as drug additives such as carriers and excipients for general formulation. In addition, in order to improve the bioavailability and stability of the koji mold fermentation product and the extract thereof according to the present invention, a drug delivery system including a formulation technology such as microcapsules, pulverization or clathration using such as cyclodextrin can be also used.

Furthermore, the above autophagy activator can be used in the forms such as tablets, granules and capsules or a liquid medicine for internal use but is preferably used in a form suitable for absorption from the digestive tracts. In addition, a conventional formulation technology can be used also when providing formulation in a form desired due to reasons such as marketability and preservability.

The autophagy activator of the present invention can be also used as a food composition in various forms such as supplement forms, e.g. tablets, capsules, granules and syrups, drinks, sweets, e.g. candy, chewing gum and chocolate, bread, rice porridge, cereal, noodle, jelly, soup, dairy products and seasonings. When the autophagy activator is used as a food composition as described above, other active ingredients, nutrients such as vitamin, mineral or amino acids and the like can be combined in various ways without affecting the effects of the active ingredient of the present invention. In food expanded from the food composition of the present invention, supplements, health food, functional food, Food for Specified Health Uses and the like are included. In addition, the daily intake of the food composition of the present invention is preferably about 10 to 1000 mg/kg body weight, more preferably about 20 to 500 mg/kg body weight and further preferably about 50 to 300 mg/kg body weight as the koji mold fermentation product P1 described above. It is also preferably about 10 to 1000 mg/kg body weight, more preferably about 20 to 500 mg/kg body weight and further preferably about 50 to 300 mg/kg body weight as the extract P2 of the koji mold fermentation product.

The present invention will now be described in more detail by way of Examples and Comparative Examples thereof. It should be noted, however, that the present invention is not limited to these Examples and Comparative Examples in any way.

### EXAMPLES

### [Example 1]

### 1. Preparation of koji mold fermentation product of tea leaves

In this Example, raw tea leaves picked from the tea plant were used as a fermentation raw material. Water was added thereto so that moisture in the tea leaves was about 50% to carry out the water absorption treatment, and the tea leaves were then put in a pressurized steam boiler for the steaming treatment. The conditions of the steaming treatment were 115°C and 60 minutes and the steaming treatment was carried out twice with one cooling therebetween. After that, the tea leaves was cooled to 30°C, and seed malt (product name: Kawachi G-type black koji from Kawachi Genichiro Shoten Co., Ltd., two billion spores/1 g of seed malt) of black koji mold (Aspergillus luchuensis var. kawachii) was then added to the tea leaves to 0.1 weight% with respect to the weight of the tea leaves, a fermentation raw material, and they were mixed, and koji was made for three days. The koji making temperature was adjusted so that the temperature of tea leaves was 35°C to 40°C for 24 hours after adding seed malt, and was adjusted slightly lower so that the temperature of tea leaves was 30°C to 35°C for the subsequent 48 hours. After that, the black koji mold fermentation product obtained by koji making for three days was subjected to a low temperature drying treatment to a water content of 8% to obtain a black koji mold fermentation product of tea leaves.

A white koji mold fermentation product of tea leaves was obtained in the same method as described above except that seed malt (product name: Kawachi white koji from Kawachi Genichiro Shoten Co., Ltd., two billion spores/1 g of seed malt) of white koji mold (Aspergillus kawachii [Aspergillus luchuensis mut. kawachii]) was used in place of the seed malt of black koji mold used to prepare the above-described black koji mold fermentation product of tea leaves.

Similarly, a yellow koji mold fermentation product of tea leaves was obtained in the same method as described above except that seed malt (product name: Kawachi yellow koji from Kawachi Genichiro Shoten Co., Ltd., two billion spores/1 g of seed malt) of yellow koji mold (Aspergillus oryzae [Aspergillus oryzae var. kawachii]) was used in place of the seed malt of black koji mold used to prepare the above-described black koji mold fermentation product of tea leaves, and the koji making temperature was adjusted so that the temperature of tea leaves was 30°C to 35°C for 24 hours after adding the seed malt, and was adjusted slightly higher so that the temperature of tea leaves was 35°C to 40°C for the subsequent 48 hours.

For comparison, raw tea leaves were treated by the same steps and methods as when using the yellow koji mold except that a koji mold was not added (seeded) to obtain a sample of tea leaves.

### [Example 2]

### 2. Preparation of extracted liquid of koji mold fermentation product of tea leaves

In order to obtain a test sample, which was added to a medium of HeLa-tfLC3 cells in the tfLC3 assay to evaluate the autophagy activity accelerating action described in Example 3 in detail, an extracted liquid of the koji mold fermentation product of tea leaves obtained in Example 1 was prepared. Because the amount of HeLa-tfLC3 cells added to the medium is set to 0.1% in the tfLC3 assay in Example 3, it is needed to prepare a test sample with a 1000-fold concentration with respect to the final concentration in the medium. Therefore, each extracted liquid sample was prepared by concentrating an extracted liquid obtained by extraction using a solvent in an evaporator.

Specifically, distilled water was used as the extraction solvent in this Example. The koji mold fermentation product of tea leaves obtained in Example 1 was crushed by a mill and 100 g of the koji mold fermentation product was added to 2000 mL of distilled water. This was left to stand in a refrigerator under conditions of 4°C to carry out an extraction treatment for 19 hours. After 19 hours, filtration was carried out using No. 2 filter paper to recover the filtrate. The recovered filtrate was concentrated using an evaporator to obtain an extracted liquid of the koji mold fermentation product of tea leaves. The results are shown in Table 1 below. It should be noted that the concentration of the koji mold fermentation product (W/W%) in Table 1 below indicates the amount of the koji mold fermentation product with respect to a mixture of distilled water, a solvent, and the koji mold fermentation product.

**[Table 1]**

| Extracted liquid sample | Koji mold fermentation product (g) | Distilled water (mL) | Concentration rate of filtrate | Concentration of koji mold fermentation product (w/w%) | Final concentration in medium in Example 3 |
|---|---|---|---|---|---|
| Black koji mold fermentation product of tea leaves | 100 | 2000 | 9.09 | 43.27% | 0.043% |
| White koji mold fermentation product of tea leaves | 100 | 2000 | 9.42 | 44.88% | 0.045% |
| Yellow koji mold fermetation product of tea leaves | 100 | 2000 | 7.24 | 34.46% | 0.034% |
| Only tea leaves (without koji mold) | 100 | 2000 | 7.20 | 34.30% | 0.034% |

### [Comparative Example 1]

### 3. Preparation of koji mold fermentation product of rice

In this Comparative Example, a koji mold fermentation product of rice, so-called "rice koji" was prepared by the same steps and methods as in Example 1 except that raw rice was used as a fermentation raw material in place of raw tea leaves used in Example 1. The same three types of koji mold as in Example 1 were used for fermentation to obtain a black koji mold fermentation product of rice, a white koji mold fermentation product of rice and a yellow koji mold fermentation product of rice. For comparison, raw rice was treated by the same steps and methods as described above except that a koji mold was not added (seeded) to obtain a rice sample.

### [Comparative Example 2]

### 4. Preparation of extracted liquid of koji mold fermentation product of rice

Distilled water was used as an extraction solvent also in this Comparative Example. The koji mold fermentation product of rice obtained in Comparative Example 1 was crushed by a mill and 100 g of the koji mold fermentation product was added to 1000 mL of distilled water. This was left to stand in a refrigerator under conditions of 4°C to carry out an extraction treatment for 19 hours. After 19 hours, filtration was carried out using No. 2 filter paper to recover the filtrate. The recovered filtrate was concentrated using an evaporator to obtain an extracted liquid of the koji mold fermentation product of rice. The results are shown in Table 2 below. It should be noted that the concentration of the koji mold fermentation product (w/w%) in Table 2 below indicates the amount of the koji mold fermentation product with respect to a mixture of distilled water, a solvent, and the koji mold fermentation product.

**[Table 2]**

| Extracted liquid sample | Koji mold fermentation product (g) | Distilled water (mL) | Concentration rate of filtrate | Concentration of koji mold fermentation product (w/w%) | Final concentration in medium in Example 3 |
|---|---|---|---|---|---|
| Black koji mold fermentation product of rice | 100 | 1000 | 3.69 | 33.53% | 0.034% |
| White koij mold fermentation product of rice | 100 | 1000 | 5.19 | 47.20% | 0.047% |
| Yellow koji mold fermentation product of rice | 100 | 1000 | 4.25 | 38.67% | 0.039% |
| Only rice (without koji mold) | 100 | 1000 | 4.74 | 43.09% | 0.043% |

### [Example 3]

### 5. Evaluation of autophagy activity accelerating action

The autophagy activity accelerating action of each extracted liquid sample adjusted in Example 2 and Comparative Example 2 described above was evaluated. Specifically, the tfLC3 assay to evaluate autophagy activity was carried out using a tfLC3 fluorescent probe in which red fluorescent protein mRFP (RFP: Red Fluorescent Protein) and green fluorescent protein EGFP (GFP: Green Fluorescent Protein) were tandemly fused to the amino terminal of LC3 (MAP1LC3: Microtubule-associated protein 1 light chain 3), an autophagosome marker protein (see Autophagy, vol. 3, pp. 452-460). The fluorescence of the GFP signal (green) of tfLC3 is attenuated under the environment in lysosome and contrarily the RFP signal (red) is stable even under the same environment. The tfLC3 assay can judge whether or not a sample added to the medium has autophagy activating ability by analyzing the GFP/RFP ratio in cells using the properties of tfLC3 which shows red by quenching of GFP when this autophagosome matures into autolysosome.

Specifically, HeLa cells having stably expressed tfLC3 (HeLa-tfLC3 cells) were established, and the HeLa-tfLC3 cells were seeded in a 96 well plate (product manufactured by PerkinElmer, product number: 6055308, PhenoPlate (registered trademark) 96-well microplates) at 3000 cells/well and cultured in a 5% CO₂ incubator set to 37°C for 24 hours. DMEM medium (product manufactured by Sigma-Aldrich, product number: D6546) supplemented with 10% FBS and 4 mM L-glutamine was used as a growing medium. After 24 hours, the growing medium was removed from each well and exchanged with a growing medium containing each extracted liquid sample at 0.1%. After the exchanges, the cells were cultured in a 5% CO₂ incubator set to 37°C for 24 hours. It should be noted that a solvent control including only a solvent used to extract and dissolve a sample (0.1% distilled water, 0.1% DMSO) and a blank control including only a growing medium (Blank) were also tested as negative controls. In addition, Torin1 which is a mTOR inhibitor and induces autophagy by blocking signals necessary to grow and proliferate the cells to imitate a state of starvation of cells was selected as a positive control substance, and the test was carried out using Torin1 dissolved in DMSO to a predetermined concentration as a positive control. After 24 hours, the cells were washed and then fixed by paraformaldehyde, and observed and analyzed under a fluorescence microscope. The test was carried out at N = 5 in each test plot and the positive control plot and at N = 15 in the negative control plot.

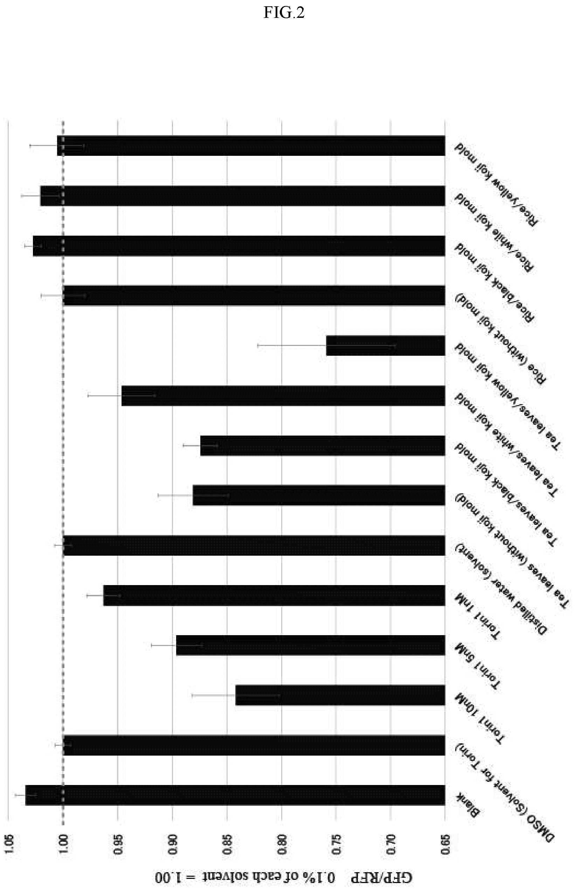

In the tfLC3 assay, when autophagosome matures into autolysosome, GFP is quenched but RFP is maintained and thus the GFP/RFP value is reduced when autophagy activity is accelerated. Therefore, the GFP/RFP value measured in the solvent control plot including only a solvent used to extract and dissolve a sample (0.1% distilled water, 0.1% DMSO) was considered 1, and the GFP/RFP value ratio measured in the test plot including each sample was obtained. The results are shown in FIG. 2 and Table 3 below.

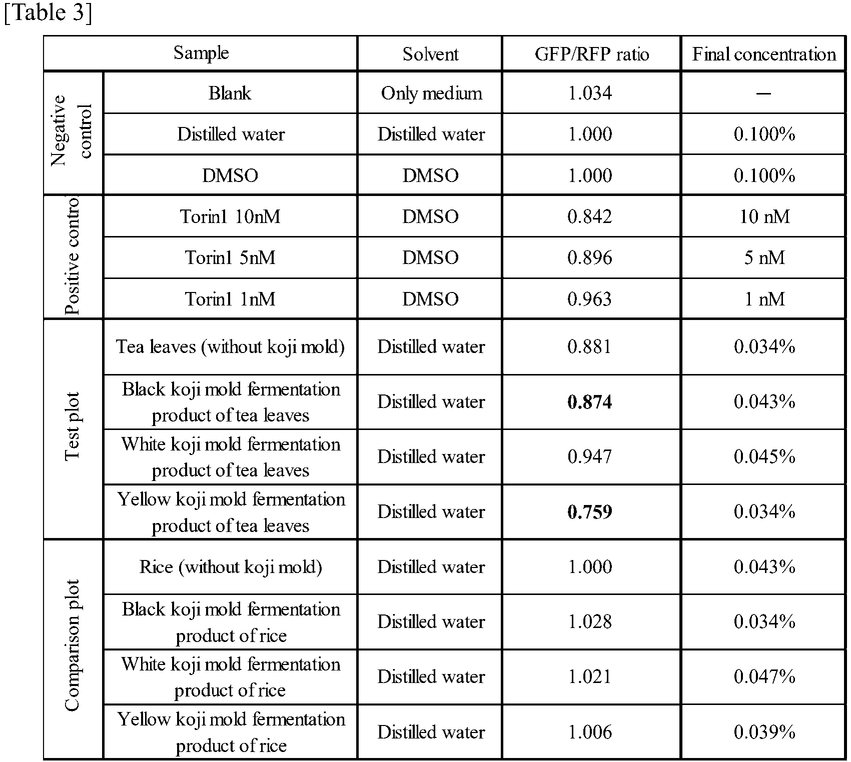

According to the results, the GFP/RFP ratio was less than 1 in all koji mold fermentation products of tea leaves and the autophagy activity accelerating effect was recognized. On the other hand, in spite of the same koji mold fermentation products, in the koji mold fermentation products of rice, the GFP/RFP ratio was about 1 in all cases, which was almost the same value as of the negative controls, and influence on autophagy activity was not recognized at all. It was found that among the koji mold fermentation products of tea leaves, the yellow koji mold fermentation product of tea leaves particularly had an GFP/RFP ratio of 0.759, which was a significant decrease rate, and had an autophagy activity accelerating action greater than of 10 nM Torin1, a positive control. In addition, the GFP/RFP ratio of the black koji mold fermentation product of tea leaves was 0.874, which showed a high decrease rate. These results showed that the koji mold fermentation product of tea leaves according to the present invention could be used as an autophagy activator to accelerate autophagy activity.

The present invention is not limited to the embodiment or examples described above and various modified design forms without departing from the spirit of the invention described in claims are also included in the technical scope.

### INDUSTRIAL APPLICABILITY

The present invention provides an autophagy activator including a koji mold fermentation product of tea leaves or an extract thereof and thus is widely useful in industries of functional food, health food, drug, medicine and the like.

## Claims

1. An autophagy activator, comprising a koji mold fermentation product of tea leaves or an extract of the koji mold fermentation product of tea leaves.

2. The autophagy activator according to claim 1, wherein the koji mold is yellow koji mold or black koji mold.

3. The autophagy activator according to claim 1 or 2, wherein the koji mold in the koji mold fermentation product is a viable koji mold.

4. The autophagy activator according to claim 1 or 2, wherein the extract of the koji mold fermentation product is a water extract or a hydrous alcohol extract of the koji mold fermentation product.

5. A method for producing an autophagy activator, comprising a step of seeding a koji mold in tea leaves or a material derived from tea leaves as a fermentation raw material, and a step of fermenting the fermentation raw material with the koji mold to obtain a koji mold fermentation product.

6. The method for producing an autophagy activator according to claim 5, wherein the koji mold is yellow koji mold or black koji mold.

7. The method for producing an autophagy activator according to claim 5 or 6, further comprising a step of extracting the koji mold fermentation product with water or a hydrous alcohol to obtain an extract of the koji mold fermentation product.

8. The method for producing an autophagy activator according to claim 7, wherein the koji mold in the koji mold fermentation product is a viable koji mold.
